# EUROPÄISCHE PATENTSCHRIFT

(11) **EP 2 412 342 B1**
(45) Veröffentlichungstag und Bekanntmachung des Hinweises auf die Patenterteilung: **02.09.2015**
(21) Anmeldenummer: 10007880.7
(22) Anmeldetag: 29.07.2010
(51) Int. Cl.: A61F 9/01, G02B 26/10

(54) **Vorrichtung zum Bearbeiten von Augengewebe mittels Femtosekundenlaserpulsen**
Device for machining eye tissue using femtosecond laser pulses
Dispositif destiné au traitement de tissu oculaire à l'aide d'impulsions laser à femtosecondes

(43) Veröffentlichungstag der Anmeldung: 01.02.2012
(73) Patentinhaber: SIE AG, Surgical Instrument Engineering, 2562 Port (CH)
(72) Erfinder: Rathjen, Christian, 28197 Bremen (DE)
(74) Vertreter: Rentsch Partner AG

(56) Entgegenhaltungen:
- EP-A1- 0 176 872
- EP-A1- 0 615 721
- US-A1- 2007 106 285
- US-B2- 7 621 637

## Beschreibung

### Technisches Gebiet

Die vorliegende Erfindung betrifft eine ophthalmologische Vorrichtung zum Bearbeiten von Augengewebe mittels Femtosekundenlaserpulsen. Die vorliegende Erfindung betrifft insbesondere eine ophthalmologische Vorrichtung mit einer Projektionsoptik zur fokussierten Projektion der Femtosekundenlaserpulse ins Augengewebe.

### Stand der Technik

Zum Bearbeiten von Augengewebe mittels Laserstrahlen wird ein Bildbereich respektive ein Arbeitsbereich mit Laserpulsen gescannt (abgetastet), indem der gepulste Laserstrahl mittels geeigneter Scannersysteme (Ablenkvorrichtungen) in ein oder zwei Scannrichtungen (Abtastrichtungen) abgelenkt wird. Die Ablenkung der Lichtstrahlen respektive der Laserpulse, beispielsweise Femtosekundenlaserpulse, wird im Allgemeinen mit beweglichen Spiegeln vorgenommen, die um eine oder zwei Scannachsen schwenkbar sind, beispielsweise mit Galvanoscannern, Piezoscannern oder Polygonscannern.

In US 7,621,637 wird eine Vorrichtung zum Bearbeiten von Augengewebe beschrieben, welche eine Basisstation mit einer Laserquelle zur Erzeugung von Laserpulsen und einen in der Basisstation angeordneten Scanner mit beweglichen Ablenkspiegeln zum Ablenken der Laserpulse in einer Scannrichtung aufweist. Die abgelenkten Laserpulse werden über ein optisches Übertragungssystem von der Basisstation zu einem Applikationskopf übertragen, der mittels eines mechanisch bewegten Lichtprojektors einen Arbeitsbereich gemäss einem Scannmuster abfährt. Die im Vergleich zur mechanischen Bewegung viel schnellere Ablenkung in der Scannrichtung wird im Applikationskopf auf die mechanische Bewegung des Lichtprojektors und somit auf dessen Scannmuster überlagert. Ein Fast-Scannersystem in der Basisstation ermöglicht eine feine Bewegung der Laserpulse (Microscann), welche auf das Scannmuster des beweglichen Lichtprojektors überlagert wird, das einen grossen Arbeitsbereich abdeckt, beispielsweise das gesamte Auge.

EP 0 176 872 beschreibt eine Vorrichtung zur Laserbehandlung der Oberfläche von Gegenständen, insbesondere zum Beschriften von Glas, mittels eines durch eine Wobbeleinrichtung gewobbelten, durch eine Ablenkvorrichtung ablenkbaren und durch ein Objektiv auf die Oberfläche des Gegenstands fokussierbaren Laserstrahls. Die Wobbeleinrichtung ist der Ablenkvorrichtung im Strahlengang vorgeschaltet und ist gemäss EP 0 176 872 vorgesehen, den Laserstrahl in der horizontalen Richtung x und in der vertikalen Richtung y mit vorgebbaren Amplituden periodisch so auszulenken, dass er auf der Oberfläche des Gegenstands einen Kreis beschreibt.

Mit der Verfügbarkeit von schnelleren Laserquellen, die zunehmend höhere Pulsraten liefern, beispielsweise über eine Million Pulse pro Sekunde (MHz), stossen die bekannten Scannersysteme an ihre physikalischen Grenzen, Pulse separiert platzieren zu können, und die Pulsrate der Laser muss künstlich reduziert werden. Insbesondere die mechanische Bewegung von Lichtprojektoren oder Linsen zum Scannen von Arbeitsbereichen, und auch die Massenträgheit von Galvanometerscannersystemen, die nicht beliebig hohe Beschleunigungen erlaubt, limitiert die möglichen Scannmuster und Scanntrajektorien in dem Sinne, dass starke Richtungsänderungen vermieden werden müssen, und dass die Pulsrate (aufwändig) aktiv reduziert werden muss oder der Laser bei Unterschreitung einer minimalen Scanngeschwindigkeit ausgeschaltet werden muss, beispielsweise in Umkehrpunkten. Somit setzen die bekannten Scannersysteme signifikante Grenzen an ausführbare Schnittführungen. Aus klinischer Sicht ist es aber gewünscht, den Schnittverlauf nach dem biomechanischen Verhalten des Gewebes zu planen und nicht unbedingt nach der Geschwindigkeit und Bandbreite des Scannersystems, wie es mit den bekannten Scannersystemen erfolgt. Im Unterschied zur Oberflächenbearbeitung kann beim Schneiden in weichem Gewebe, beispielsweise Augengewebe, nicht immer mit einfachen Pulsrastern, beispielsweise Zeilen- oder Spiralmuster, gearbeitet werden, da durch interne Gasentwicklung oder Freiwerden von Spannungen Gewebedeformationen verursacht werden können, die es durch geeignet komplexere Scann- respektive Abtastmuster unter Berücksichtigung des erwarteten biomechanischen Verhaltens des Gewebes zu vermeiden gilt. Die bekannten Scannersysteme ermöglichen zwar die Bearbeitung von einfachen Scannmustern, beispielsweise das Schneiden eines Gewebelappens (Flap), das in der Regel als grosses Flächenstück mit einfacher Randgeometrie ausgeführt wird. Bei vereinzelten Bearbeitungsgebieten mit komplizierter Randgeometrie, wie sie zum Beispiel zur refraktiven Korrektur benötigt werden, oder bei anderen, biomechanisch bedingten komplexeren Scannrespektive Abtastmustern, ist das allerdings nicht mehr so einfach möglich. Beispielsweise muss dann ein grossflächiges Scannmuster mit einer Maske belegt werden (elektronisch oder optisch), oder die kleinen Bereiche werden einzeln bearbeitet, z.B. abgerastert, was eine entsprechende Reduktion der Verarbeitungsgeschwindigkeit zur Folge hat, da das Scannersystem bezogen auf die zu scannende Strecke sehr häufig abbremsen und beschleunigen muss.

### Darstellung der Erfindung

Es ist eine Aufgabe der vorliegenden Erfindung eine ophthalmologische Vorrichtung zum Bearbeiten von Augengewebe mittels Femtosekundenlaserpulsen vorzuschlagen, welche zumindest einige Nachteile der bekannten Systeme nicht aufweist. Es ist insbesondere eine Aufgabe der vorliegenden Erfindung eine ophthalmologische Vorrichtung zum Bearbeiten von Augengewebe mittels Femtosekundenlaserpulsen vorzuschlagen, welche ohne mechanische Bewegung von Linsen für das Scannen eines Bearbeitungsgebiets auskommt und den Einsatz von Laserquellen mit hohen Pulsfrequenzen, insbesondere Pulsfrequenzen im MHz-Bereich mit über einer Million Pulsen pro Sekunde, zur Reduzierung der Bearbeitungszeit und eine flexiblere Schnittführung ermöglicht.

Gemäss der vorliegenden Erfindung werden diese Ziele durch die Merkmale des Anspruchs 1 erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass eine ophthalmologische Vorrichtung zum Bearbeiten von Augengewebe, insbesondere über das gesamte Auge, mittels Femtosekundenlaserpulsen, welche eine Projektionsoptik zur fokussierten Projektion der Femtosekundenlaserpulse ins Augengewebe umfasst, zudem versehen ist mit einem ersten der Projektionsoptik vorgelagerten strahlablenkenden Scannersystem zum Scannen des Augengewebes mit den Femtosekundenlaserpulsen entlang einer Bearbeitungslinie, einem zweiten dem ersten Scannersystem vorgelagerten strahlablenkenden Scannersystem zum Scannen des Augengewebes mit den Femtosekundenlaserpulsen in einer der Bearbeitungslinie überlagerten, in einer Ablenkungsebene verlaufenden Scannbewegung, und einem Rotationssystem zum Ausrichten der Ablenkungsebene mit einem definierten Winkel zur Bearbeitungslinie.

Aus gerätetechnisch praktischen Gründen und aus Kostengründen ist es erforderlich, die Grösse (z.B. der Durchmesser) der Projektions- und Übertragungsoptiken so zu begrenzen, dass in der Regel Auslenkungs- respektive Scannwinkel von deutlich kleiner als 90 Grad sinnvoll realisierbar sind. Bei sehr kleinem Fokus der Laserpulse (Spots), wie sie für einen gewebeschonende und präzise Bearbeitung erforderlich sind, beispielsweise Spots mit einem Durchmesser kleiner als 5µm, insbesondere kleiner als 3µm, vorzugsweise kleiner als 1µm, und grossem Bildfeld (Bearbeitungsbereich) müssen daher grosse Strahldurchmesser gescannt werden, was grosse schwere Spiegel und damit, wie bei Lösungen mit mechanisch bewegten Linsen, geringe Scannfrequenzen bedingt. Mit anderen Worten: Grosse Bildfelder können aus fundamentalen physikalischen Gründen nicht mit kleinen (d.h. schnell ablenkbaren) Spiegeln gescannt werden und gleichzeitig für die Bearbeitungsqualität wünschenswert kleine Spotdurchmesser haben.

Durch das Hintereinanderschalten (Kaskadierung) der zwei strahlablenkenden Scannersystem mit beweglichen Ablenkspiegeln wird eine flexibel konfigurier- und steuerbare Bearbeitung von Augengewebe ermöglicht, wobei das erste Scannersystem ein ausgedehntes Bearbeitungsgebiet abdeckt, beispielsweise das gesamte Auge, und das vorgeschaltete zweite Scannersystem eine schnelle feine Scannbewegung überlagert, deren Form, Grösse und Ausrichtung flexibel einstellbar ist, ohne dass übergrosse Projektions- und

Übertragungsoptiken eingesetzt werden müssen, und ohne dass für die Abtastung eine mechanische Bewegung von Linsen oder Projektionsobjektiven erforderlich ist, so dass hohe Scannfrequenzen respektive Abtastraten realisierbar sind. Die Anordnung des vergleichsweise schnelleren zweiten Scannersystem, das "ultraschnelle Scannersystem", zwischen der Laserquelle und dem ersten Scannersystem, das "schnelle Hauptscannersystem", erlaubt die Verwendung kleinerer Strahlaperturen, z.B. Spiegel, und ermöglicht damit ein schnelles, hochfrequentes Scannen des Augengewebes. Dabei kann das schnellere zweite Scannersystem auf hochfrequentes Abtasten und Bearbeiten, d.h. Schneiden, des Augengewebes mit relativ kleiner Auslenkung optimiert werden, und das erste Scannersystem auf das schnelle Anfahren von beliebig adressierbaren Punkten im ausgedehnten Bearbeitungsgebiet (Bildbereich). Durch die automatische Ausrichtung der Ablenkungsebene und dadurch der feinen Scannbewegung des ultraschnellen Scannersystems zur Bearbeitungslinie des Hauptscannersystems, beispielsweise normal oder in einem anderen definierten Winkel, werden komplexe Schnittformen ermöglicht, die nicht auf rasterförmige Scannmuster begrenzt sind.

Vorzugsweise weist das erste Scannersystem zwei orthogonal zueinander ausgerichtete Scannachsen zum Ablenken der Femtosekundenlaserpulse auf, das zweite Scannersystem ist eingerichtet, die Femtosekundenlaserpulse mit einer Scanngeschwindigkeit abzulenken, die ein Vielfaches der Scanngeschwindigkeit des ersten Scannersystems beträgt, und das Rotationssystem ist dem ersten Scannersystem vorgelagert.

Bei spiegelbasierten Scannersystemen ist der Begriff Scannachse als gleichwertig zur Spiegelachse zu verstehen, so dass eine Auslenkung des Spiegels um die Scannachse eine Ablenkung eines Laserstrahls in einer in der Ablenkungsebene verlaufende Scannrichtung bewirkt. Bei anderen Scannersystem, welche keine Spiegelachse aufweisen, ist der Begriff Scannachse als virtuelle Achse zu verstehen, um die ein Spiegel gedreht werden müsste, um den Laserstrahl in die betreffende Scannrichtung abzulenken.

Erfindungsgemäss umfasst das Rotationssystem ein Rotationsmodul und ein Rotationssteuermodul, wobei das Rotationsmodul eingerichtet ist, eine definierte Drehung der Ablenkungsebene um eine optische Projektionsachse auszuführen, und wobei das Rotationssteuermodul eingerichtet ist, die Drehung der Ablenkungsebene basierend auf dem Verlauf der Bearbeitungslinie zu definieren.

Je nach Ausführungsvariante umfasst das Rotationsmodul ein aus Spiegeln ausgestaltetes Rotationselement, ein aus Prismen ausgestaltetes Rotationselement, und/oder ein mit dem zweiten Scannersystem gekoppeltes Antriebsmodul, welches eingerichtet ist das zweite Scannersystem um die optische Projektionsachse zu drehen.

In einer Ausführungsvariante weist das zweite Scannersystem zwei orthogonal zueinander ausgerichtete Scannachsen zum Ablenken der Femtosekundenlaserpulse auf und das Rotationssteuermodul ist eingerichtet, die Ausrichtung der in der Ablenkungsebene verlaufenden Scannbewegung zur Bearbeitungslinie durch gekoppelte Steuerung von der Auslenkungsamplitude der Ablenkung um die erste Scannachse und von der Auslenkungsamplitude der Ablenkung um die zweite Scannachse zu bestimmen. Durch die einfache Steuerung der Ausrichtung der Scannbewegung basierend auf den Auslenkungsamplituden wird ein kostspieliger und langsamer mechanischer und/oder optischer Bildrotator hinfällig, da die Steuerung gänzlich über elektronische und/oder programmtechnische Mittel erfolgt.

In einer Ausführungsvariante umfasst die Vorrichtung ein Scannsteuermodul, das eingerichtet ist, die Breite der Scannbewegung des zweiten Scannersystem abhängig vom Richtungsverlauf der Bearbeitungslinie und/oder von der aktuellen Scanngeschwindigkeit des ersten Scannersystems zu steuern. Beispielsweise wird die Breite der Scannbewegung bei langsamer Vorschubgeschwindigkeit in Richtung der Bearbeitungslinie erhöht, um dadurch den Abstand zwischen einzelnen projizierten Femtosekundenlaserpulsen zu erhöhen respektive die Anzahl der Femtosekundenlaserpulse innerhalb einer definierten Scannbreite zu reduzieren. Das Scannsteuermodul ist also eingerichtet, die Breite der Scannbewegung des zweiten Scannersystem so zu steuern, dass der Abstand zwischen zwei aufeinanderfolgenden Femtosekundenlaserpulsen und die Anzahl der Femtosekundenlaserpulse in einem definierten Durchlassbereich variierbar ist.

In einer weiteren Ausführungsvariante ist zwischen dem ersten Scannersystem und dem zweiten Scannersystem eine Blende angeordnet zum Ausblenden von Femtosekundenlaserpulsen, die vom vorgelagerten zweiten Scannersystem in einen Bereich ausserhalb eines definierten Durchlassbereichs respektive einer definierten Scannbreite abgelenkt werden.

In einer weiteren Ausführungsvariante weist die Blende einen steuerbaren, variablen Blendenbereich respektive Durchlassbereich auf.

In einer Ausführungsvariante umfasst die Vorrichtung ein Blendensteuermodul, das eingerichtet ist, die Grösse des variablen Blendenbereichs respektive Durchlassbereichs abhängig von der Scanngeschwindigkeit des ersten Scannersystems zu steuern.

Vorzugsweise ist die Blende als Feldblende ausgestaltet.

Vorzugsweise weist das erste Scannersystem einen im Vergleich zum zweiten Scannersystem wesentlich grösseren Auslenkungsgrad auf.

In einer Ausführungsvariante sind die zwei orthogonal zueinander ausgerichteten Scannachsen des ersten Scannersystems mit einem gemeinsamen Ablenkspiegel gekoppelt,

In einer Ausführungsvariante ist das zweite Scannersystem eingerichtet, das Augengewebe mit den Femtosekundenlaserpulsen in einer oszillierenden Scannbewegung zu scannen.

In einer Ausführungsvariante umfasst die Vorrichtung ein steuerbares Filtermodul zum selektiven Ausschliessen von Femtosekundenlaserpulsen in einem definierten Bereich der überlagerten Scannbewegung.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1:: zeigt ein Blockdiagramm, welches schematisch eine ophthalmologische Vorrichtung mit zwei hintereinandergeschalteten Scannersystemen und einem Rotationssystem zum Bearbeiten von Augengewebe mittels Femtosekundenlaserpulsen illustriert.
- Figur 1a:: zeigt schematisch in einer Projektionsebene die Überlagerung von nicht phasenverschoben schwingenden Scannbewegungen um zwei orthogonal zueinander angeordnete Spiegel- oder Scannachsen.
- Figur 1b:: zeigt schematisch eine Bearbeitungsebene mit einer aus der Hintereinanderschaltung der Scannersysteme resultierenden Scanntrajektorie.
- Figur 2:: illustriert die Überlagerung einer Scannbewegung eines Scannersystems auf die Bearbeitungslinie eines weiteren Scannersystems, wobei bei einer Richtungsänderung der Bearbeitungslinie die Ausrichtung der Scannbewegung zur Bearbeitungslinie automatisch angepasst wird.
- Figur 3:: illustriert eine Sequenz von Zwischenstufen bei einer kontinuierlichen Vergrösserung respektive Verkleinerung der Auslenkungsamplituden der Ablenkungen um zwei orthogonal zueinander ausgerichtete Spiegel- oder Scannachsen zur dynamischen Anpassung der Ausrichtung der Scannbewegung an eine Richtungsänderung der Bearbeitungslinie.
- Figur 4:: illustriert die Überlagerung einer Scannbewegung eines Scannersystems auf die Bearbeitungslinie mit definierter Ausrichtung der Scannbewegung zur Bearbeitungslinie und ausgeblendeten Schwingungsspitzen der resultierenden Scanntrajektorie.
- Figur 5:: illustriert die Überlagerung einer Scannkurve eines Scannersystems auf die Bearbeitungslinie eines weiteren Scannersystems, wobei zur Erzeugung eines begradigten Scannmusters jeweils die aufsteigenden Flanken der Scannkurve ausgeblendet werden.

### Wege zur Ausführung der Erfindung

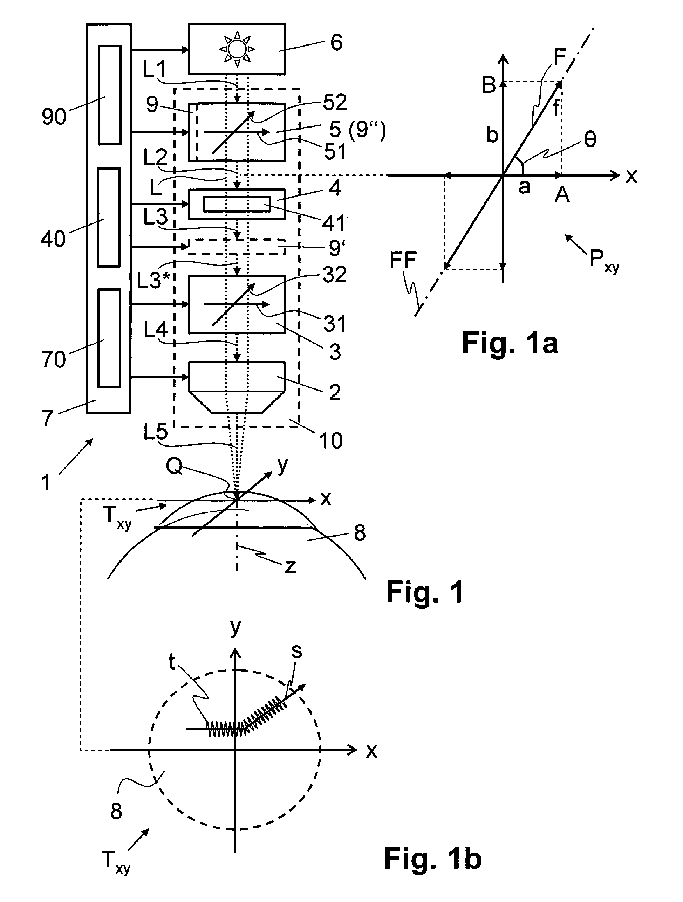

In der Figur 1 bezieht sich das Bezugszeichen 1 auf eine ophthalmologische Vorrichtung zum Bearbeiten von Augengewebe 8 mittels eines gepulsten Laserstrahls L1 mit Femtosekundenlaserpulsen. Der gepulste Laserstrahl L1, vorzugsweise mit Pulsfrequenzen im MHz-Bereich mit über einer Million Pulsen pro Sekunde, wird von einer Strahlquelle 6 geliefert und über ein optisches Übertragungssystem 10 fokussiert entlang einer Scanntrajektorie t als gepulster Bearbeitungsstrahl L5 auf, respektive in das Augengewebe 8 projiziert. Die Strahlquelle 6 ist je nach Ausführung Bestandteil des optischen Übertragungssystems 10 oder als separate Einheit ausgestaltet, welche über ein Lichtübertragungssystem, beispielsweise eine Lichtfaserleitung und/oder ein Spiegel-/Linsensystem, mit dem optischen Übertragungssystem 10 verbunden ist.

Wie in der Figur 1 schematisch dargestellt ist, umfasst die ophthalmologische Vorrichtung 1 respektive das optische Übertragungssystem 10 zwei strahlablenkende Scannersysteme 3, 5 und ein optionales Filtermodul 4, die im Strahlengang (L1-L2-L3-L3*-L4) von der Strahlquelle 6 zur Projektionsoptik 2 angeordnet sind.

Das strahlablenkende Scannersystem 5 weist eine Scanngeschwindigkeit auf, die ein Vielfaches der Scanngeschwindigkeit des Scannersystems 3 beträgt. Entsprechend werden in der nachfolgenden Beschreibung das Scannersystem 5 als Fast-Scannersystem 5 und das Scannersystem 3 als Slow-Scannersystem 3 bezeichnet. Das Fast-Scannersystem 5 weist somit eine um ein Vielfaches schnellere Ablenkgeschwindigkeit und, bei oszillierenden Scannersystemen, um ein Vielfaches höhere Oszillationsfrequenzen (ωₓ, ω_{y}) auf, als mit dem Slow-Scannersystem 3 durchführbar wären. Insbesondere weist das Fast-Scannersystem 5 eine höhere Scanngeschwindigkeit im Bildfeld des Projektionsobjektivs (Schneidobjektiv) auf, also im Bildfeld der Projektionsoptik 2, und/oder hat eine höhere Scannfrequenz. Andererseits weist das Slow-Scannersystem 3 einen im Vergleich zum Fast-Scannersystem 5 wesentlich grösseren Auslenkungsgrad im Bildfeld der Projektionsoptik auf. Das Slow-Scannersystem 3 ermöglicht somit ein im Vergleich zum Fast-Scannersystem 5 viel grösseres Bildfeld und Bearbeitungsgebiet abzudecken und anzusteuern, so dass das Augengewebe 8 über das gesamte Auge vollständig gescannt und mit Femtosekundenlaserpulsen bearbeitet werden kann.

Das Fast-Scannersystem 5 ist vorzugsweise zwischen der Strahlquelle 6 und dem Slow-Scannersystem 3 angeordnet. Das Fast-Scannersystem 5 ist beispielsweise im Strahlengang unmittelbar nach der Strahlquelle 6 angeordnet.

Das Slow-Scannersystem 3 ist vorzugsweise zwischen dem Fast-Scannersystem 5 und der Projektionsoptik 2 angeordnet. Das Slow-Scannersystem 3 ist beispielsweise im Strahlengang unmittelbar vor der Projektionsoptik 2 angeordnet.

Das Fast-Scannersystem 5 weist je nach Ausführungsvariante eine oder zwei Scannachsen (Spiegelachsen) 51, 52 auf, wobei letztere vorzugsweise orthogonal zueinander ausgerichtet sind. Die Scannachsen 51, 52, die auch als Scannachsen bezeichnet werden, sind je nach Ausführungsvariante mit einem gemeinsamen, verkippbaren (Tip-Tilt-Modus) Ablenkungsspiegel oder mit jeweils einem eigenen, separaten Ablenkungsspiegel gekoppelt, welche hintereinandergeschaltet angeordnet sind. Die Verwendung eines Scannersystems mit zwei Scannachsen 51, 52 und einem gemeinsamen verkippbaren Ablenkungsspiegel hat den Vorteil, dass sich kostspielige Zwischenoptiken einspaaren lassen und der gesamte Aufbau der Vorrichtung 1 kompakter ausfällt. Das Fast-Scannersystem 5 lenkt den gepulsten Laserstrahl L1 von der Strahlquelle 6 respektive dessen Femtosekundenlaserpulse mit einer definierten Scannbewegung f in einer Ablenkungsebene FF ab, die zu einer in der Figur 1a mit der Zeichenebene übereinstimmenden Projektionsebene P_{xy} normal ausgerichtet ist. In der in Figur 1a schematisch dargestellten Projektionsebene P_{xy} des Fast-Scannersystems 5, sind die Scannachse 51 entlang der x-Achse und die Scannachse 52 entlang der y-Achse ausgerichtet. Der Bewegungsvektor a stellt die Auslenkung des Ablenkungsspiegels um die Scannachse 52 (y-Achse) mit einer Auslenkungsamplitude A in x-Richtung, und der Bewegungsvektor b stellt die Auslenkung des Ablenkungsspiegels um die Scannachse 51 (x-Achse) mit einer Auslenkungsamplitude B in y-Richtung dar. Bei einer synchronisierten, d.h. gleichfrequentig (ωₓ=ω_{y}) und nicht-phasenverschobenen (ϕ=0), Auslenkung um die beiden Scannachsen 51, 52 werden die Femtosekundenlaserpulse mit einer Scannbewegung f abgelenkt, die in Figur 1a durch einen entsprechenden Bewegungsvektor dargestellt ist. Die Auslenkungsamplituden A, B bestimmen somit die Ausrichtung θ und Auslenkungsamplitude F der resultierenden Scannbewegung f des Scannersystems 5 in der zur Projektionsebene P_{xy} normalen Ablenkungsebene FF.

Das Slow-Scannersystem 3 weist vorzugsweise zwei orthogonal zueinander ausgerichtete Scannachsen (Spiegelachsen) auf, die mit einem gemeinsamen, verkippbaren Ablenkspiegel oder mit zwei separaten Ablenkspiegein gekoppelt sind.

Vorzugsweise ist das Slow-Scannersystem 3 frei adressierbar in Form von zwei Galvanometer-Scannern oder mit einem im Tip-Tilt-Modus verkippbaren zweiachsigen Ablenkungsspiegel Spiegel ausgeführt (z.B. über Piezoelemente angesteuert). Je nach Betriebsmodus oder Konstruktion ist das Fast-Scannersystem 5 als resonanter, oszillierender, oder frei adressierbarer Scanner ausgeführt. Ein sinusförmiger Betriebsmodus erlaubt insbesondere bei mechanischen Resonanzscannern mit Schwingspiegeln (auch als MEM (Micro Electro-Mechanical) Scanner oder mit Piezo-Antrieb) höhere Frequenzen und Ablenkgeschwindigkeiten als mit Galvanometer-Scannern möglich sind. Deshalb werden bevorzugt oszillierende Fast-Scannersysteme 5 im resonanten Betriebsmodus eingesetzt, da diese auf Grund ihrer hohen Scannfrequenzen für den praktischen Einsatz besonders vorteilhaft sind. Weitere Scannertypen, die zum Teil noch höhere Frequenzen erlauben, sind aus der Literatur bekannt (z.B. AOM (Acousto-optic Modulator) Scanner oder EOM (Electrooptic Modulator)). Vorzugsweise werden das Fast-Scannersystem 5 und das Slow-Scannersystem 3 mit orthogonalen Scannachsen (Spiegelachsen) ausgeführt und betrieben.

Das optionale Filtermodul 4 ist im Strahlengang vorzugsweise zwischen dem Fast-Scannersystem 5 und dem Slow-Scannersystem 3 angeordnet. Je nach Ausführungsvariante umfasst das Filtermodul 4 eine feste und/oder steuerbare Blende 41, die auch als Shutter ausgeführt sein kann, wobei der Shutter vorzugsweise beim Laser angeordnet und unmittelbar der Strahlquelle 6 nachgeschaltet ist. In einer Ausführungsvariante ist die Blende 41 als Feldblende ausgeführt, also in einer Zwischenbildebene angeordnet, wobei überdies je nach Ausführungsvariante die Feldblende variabel und/oder asymmetrisch ausgestaltet ist.

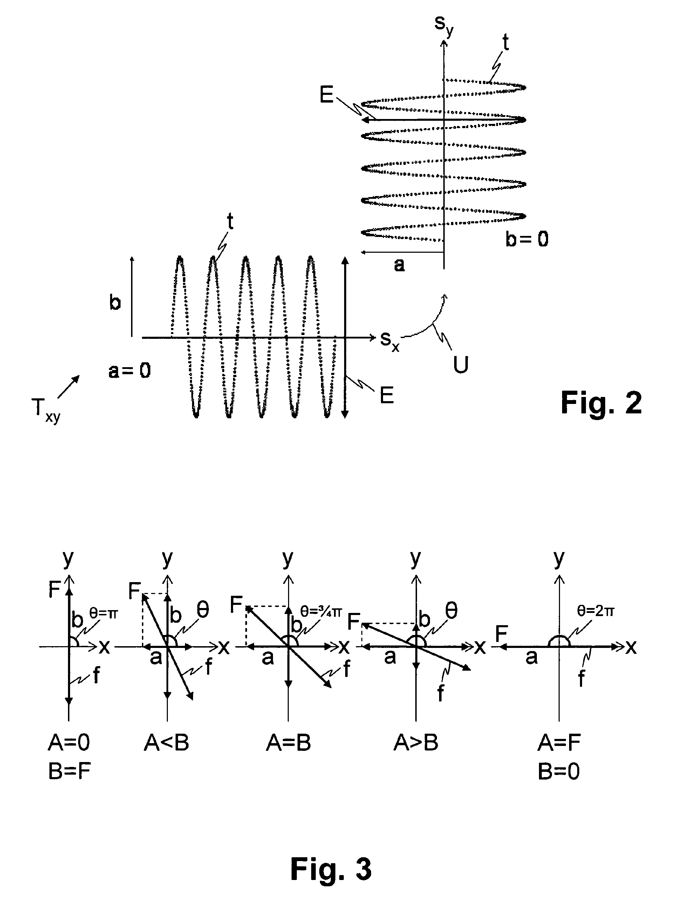

Das Slow-Scannersystem 3 ist eingerichtet das Augengewebe 8 mit den Femtosekundenlaserpulsen in einem ausgedehnten Bearbeitungsbereich entlang einer Bearbeitungslinie s zu scannen. Dabei lenkt das Slow-Scannersystem 3 die vom Fast-Scannersystem 5 abgelenkten Femtosekundenlaserpulse L2 respektive die vom Filtermodul 4 gefilterten und nicht ausgeblendeten Femtosekundenlaserpulse L3, L3* ab. Die Richtung und Form der Bearbeitungslinie s wird wie obenstehend mit Bezug zu Figur 1a für das Fast-Scannersystem 5 beschrieben durch die Auslenkungsamplituden der Auslenkungen um die Scannachsen (Spiegelachsen) 31, 32 des Slow-Scannersystems 3 bestimmt. Die durch das Fast-Scannersystem 5 erzeugte Scannbewegung f wird somit der durch das Slow-Scannersystem 3 kontinuierlich gescannten Bearbeitungslinie s überlagert, wodurch in der Bearbeitungsebene T_{xy}, wie in Figur 1b dargestellt, eine resultierende Scanntrajektorie t gebildet wird, mit der das Augengewebe 8 tatsächlich bearbeitet wird. In den Figuren 2, 4 und 5 sind Beispiele verschiedener erzeugter Scanntrajektorien t zur Bearbeitung des Augengewebes 8 auf einer Bearbeitungsebene T_{xy} dargestellt, auf die später detaillierter eingegangen wird.

Die ophthalmologische Vorrichtung 1 umfasst zudem ein Rotationssystem zum Ausrichten der Ablenkungsebene FF mit einem definierten Ausrichtungswinkel zur Bearbeitungslinie s, beispielsweise normal (π/2) oder in einem anderen definierten Winkel. Durch das Rotationssystem wird somit sichergestellt, dass die Scannbewegung f auch bei Richtungsänderungen in der Bearbeitung definiert auf die Bearbeitungslinie s ausgerichtet ist. Das Rotationssystem umfasst ein im optischen Übertragungssystem 10, im Strahlengang (L1-L2-L3-L3*-L4) von der Strahlquelle 6 zur Projektionsoptik 2, angeordnetes Rotationsmodul 9, 9', 9" zum Ausführen einer definierten Drehung der Ablenkungsebene FF, und der darin ausgeführten Scannbewegung f, um eine optische Projektionsachse z. In verschiedenen Ausführungsvarianten ist das Rotationsmodul 9 als mechanisches Antriebsmodul ausgeführt, das mit dem Fast-Scannersystem 5 gekoppelt ist, um das Fast-Scannersystem 5 und damit die Ablenkungsebene FF um die optische Projektionsachse z zu drehen, oder das Rotationsmodul 9' umfasst ein aus Prismen oder Spiegeln ausgestaltetes Rotationselement, beispielsweise ein drehbarer K-Spiegel, und ein Antriebsmodul, das mit dem Rotationselement gekoppelt ist und dieses und damit die Ablenkungsebene FF um die optische Projektionsachse z dreht, oder die Funktion des Rotationsmoduls 9" wird durch das Fast-Scannersystem 5 respektive den/die um die zwei Scannachsen 51, 52 drehbaren Ablenkspiegel des Fast-Scannersystems 5 ausgeführt. Bei den Ausführungsvarianten, in denen das Rotationsmodul 9, 9' ein mechanisches Antriebsmodul umfasst, kann das Fast-Scannersystem 5 für andere Funktionen als die definierte Ausrichtung der Ablenkungsebene F zur Bearbeitungslinie s verwendet werden, beispielsweise für die Erzeugung von Scanbewegungen f, welche durch unterschiedliche Ausrichtungen θ, Amplituden F und ggf. Formen definiert sind, wie später detaillierter beschrieben wird.

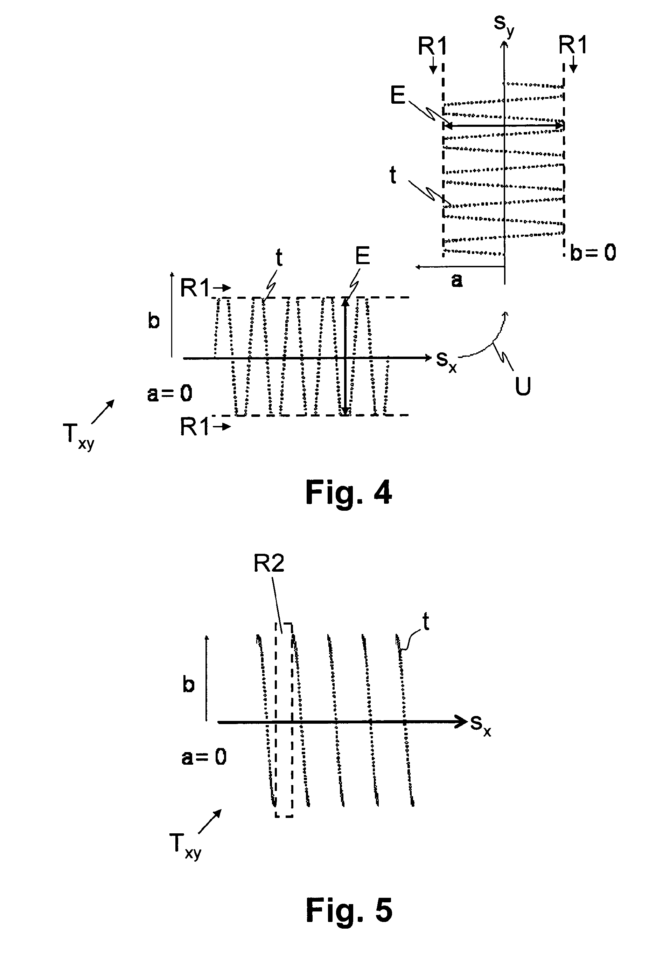

Das Filtermodul 4 ist eingerichtet, gewisse der vom Fast-Scannersystem 5 abgelenkten Femtosekundenlaserpulse L2 gemäss definierten Filterkriterien auszublenden. Die Blende 41 ist beispielsweise fest oder steuerbar eingerichtet, Femtosekundenlaserpulse L2, die vom Fast-Scannersystem 5 in einen Bereich ausserhalb einer definierten Scannbreite E abgelenkt werden auszublenden, oder abgelenkte Femtosekundenlaserpulse L2 vollständig oder in einem definierten Bereich R1, R2 der Scannkurve f auszuschliessen. Im Beispiel der Figur 4 ist das Filtermodul 4 eingerichtet, die Spitzen der Scannbewebung f, beispielsweise bei Auslenkungsamplituden F über einem definierten Amplitudenwert respektive Scannbreite E, im Bereich R1 auszublenden. Im Beispiel der Figur 5, ist das Filtermodul 4 eingerichtet, jeweils die aufsteigenden Flanken der Scanntrajekorie t im Bereich R2 auszublenden, so dass ein Scannmuster mit nahezu parallelen Scannstrecken erzeugt wird. Die Ausblendbereiche R1, R2 sind beispielsweise dynamisch und frei definier- und veränderbar, beispielsweise abhängig von den Auslenkungen a, b um die Scannachsen 51, 52 und/oder von der Richtung der Bearbeitungslinie s.

Die Projektionsoptik 2 ist eingerichtet, die vom Slow-Scannersystem 3 abgelenkten Femtosekundenlaserpulse L4 fokussiert auf respektive in das Augengewebe 8 zu projizieren, wobei das Augengewebe 8 im Fokuspunkt Q, abhängig von der Scanngeschwindigkeit respektive Vorschubgeschwindigeit in Richtung der Bearbeitungslinie s, jeweils durch einen einzelnen Femtosekundenlaserpuls oder durch mehrere nacheinander überlappend projizierte Femtosekundenlaserpulse aufgelöst wird. In einer Ausführungsvariante ist die Projektionsoptik 2 zudem eingerichtet den Fokus Q des fokussierten, abgelenkten, gepulsten Laserstrahls L5, beispielsweise durch vertikale Verschiebungen, in der Projektionsrichtung, einzustellen. Ansonsten ist die Projektionsoptik 2 während der Behandlung stationär, d.h. für die Abtastung und Bearbeitung des Augengewebes 8 bedarf es keiner lateralen mechanischen Bewegung (in x- und/oder y-Richtung) von Linsen der Projektionsoptik 2, nachdem diese für die geplante Behandlung auf das Auge des Patienten ausgerichtet wurde. Eine Fixierung am Auge erfolgt beispielsweise mittels eines vakuumgesteuerten Saugrings.

Wie in der Figur 1 ersichtlich ist, umfasst die ophthalmologische Vorrichtung 1 ein Steuermodul 7, das je nach Ausführung als Bestandteil des optischen Übertragungssystems 10 oder als separate Einheit ausgestaltet ist, welche über Steuerleitungen respektive eine oder mehrere Datenkommunikationsverbindungen, beispielsweise mehrere Signal- und/oder Datenleitungen und/oder ein Datenbus, zur Steuerung mit dem optischen Übertragungssystem 10 verbunden ist. Das Steuermodul 7 ist je nach Ausführung mit der Strahlquelle 6, dem Fast-Scannersystem 5, dem Filtermodul 4, dem Rotationsmodul 9, 9', 9", dem Slow-Scannersystem 3 und/oder der Projektionsoptik 2 verbunden. Wie in der Figur 1 schematisch dargestellt ist umfasst das Steuermodule 7 mehrere Funktionsmodule nämlich ein Rotationssteuermodul 90, ein Scannsteuermodul 70 und ein Blendensteuermodul 40. Das Steuermodul 7 umfasst vorzugsweise einen oder mehrere Prozessoren und einen greifbaren computerlesbaren Datenträger (Computerprogrammprodukt), welcher fest oder entfernbar mit den Prozessoren verbunden ist und auf welchem mindestens ein programmiertes Softwaremodul gespeichert ist, das Computerprogrammcode zur Steuerung der Prozessoren umfasst. Der Fachmann wird verstehen, dass das Steuermodul 7 respektive seine Funktionsmodule in verschiedenen Ausführungsvarianten als programmierte Softwaremodule oder aber vollständig oder wenigstens teilweise mit Hardwarekomponenten implementiert sind.

In den folgenden Abschnitten wird die Funktionalität des Steuermoduls 7 respektive seiner Funktionsmodule und die dadurch bewirkte Steuerung der Prozessoren und damit der ophthalmologischen Vorrichtung 1 mit Bezug zu den Figuren 2, 3, 4 und 5 beschrieben.

Je nach Ausführungsvariante ist das Steuermodul 7 eingerichtet, die Strahlquelle 6 und/oder die Projektionsoptik 2 zu steuern, beispielsweise hinsichtlich Plusenergie, Pulsfrequenz respektive Fokustiefe, was jedoch in den nachfolgenden Abschnitten nicht detaillierter beschrieben werden soll.

Das Scannsteuermodul 70 umfasst insbesondere eine Fastscannersteuerung zum Steuern des Fast-Scannersystems 5. Die Fastscannsteuerung ist eingerichtet, die Amplitude F respektive Scannbreite der Scannbewegung f des Fast-Scannersystems 5 zu steuern. Das Scannsteuermodul 70 bestimmt dadurch den Abstand zwischen zwei aufeinanderfolgenden abgelenkten Femtosekundenlaserpulsen in der Projektionsebene P_{xy} respektive die Anzahl von Femtosekundenlaserpulsen innerhalb einer definierten Scannbreite E. Die Fastscannsteuerung ist vorzugsweise eingerichtet, die Amplitude F respektive Breite der Scannbewegung f und damit die Pulszahl innerhalb der Scannbreite abhängig vom Richtungsverlauf der Bearbeitungslinie s und/oder abhängig von der aktuellen Scanngeschwindigkeit des Slow-Scannersystems 3 (respektive Vorschubgeschwindigeit in Richtung der Bearbeitungslinie s) zu steuern. Bei der Ausführung des Fast-Scannersystems 5 mit zwei Scannachsen 51, 52 ist die Fastscannsteuerung eingerichtet, die Ablenkung a, b der Femtosekundenlaserpulse um die Scannachsen 51, 52, wie in der Tabelle 1 angegeben, zur Ausführung definierter Scannbewegungen f zu steuern, welche durch unterschiedliche Ausrichtungen θ, Amplituden F und ggf. Formen definiert sind (neben geraden sind auch Scannbewegungen f erzeugbar, die Formen mit elliptischen, Lissajous-, sinus-, sägezähn-, trapez- oder rechteckförmigen Schwingungen oder anderen Formen aufweisen, die sich über Fouriersynthese herstellen lassen). Um die Femtosekundenlaserpulse gemässs einer definierten Scannbewegung f abzulenken verwendet das Steuermodul 7 entsprechende Steuerparameter zur Steuerung des Fast-Scannersystems 5, die der betreffenden mit einer Kennung bezeichneten Scannbewegung f zugeordnet sind. Die Auslenkungen um die Scannachsen 51, 52 werden insbesondere mit Parametern zur Steuerung der jeweiligen Auslenkungsamplituden A, B, Auslenkungsfrequenz ωₓ, ω_{y} und/oder relative Phase ϕ (Phasenverschiebung) zwischen den Auslenkungen a, b (Oszillation) um die Scannachsen 51, 52 bestimmt.

**Tabelle 1**

| Scannbewegung | | | | Steuerparameter | | | | |
|---|---|---|---|---|---|---|---|---|
| | | | | Auslenkung um erste Scannachse | | Auslenkung um zweite Scannachse | | Phasenverschiebung |
| Kennung | Form | Ausrichtung | Amplitude | Amplitude | Frequenz | Amplitude | Frequenz | |
| ... | ... | ... | ... | ... | ... | ... | ... | ... |

Figur 1 a illustriert ein Beispiel wie durch Steuerung der Auslenkungsamplituden A, B um die Scannachsen 51, 52 die Ausrichtung θ und Auslenkungsamplitude F der resultierenden Scannbewegung f auf der Projektionsebene P_{xy} durch das Scannsteuermodul 70 einstellbar sind, wenn die verbleibenden Steuerparameter so gesetzt werden, dass die Ablenkung a, b um die beiden Scannachsen 51, 52 mit gleicher Frequenz ωₓ=ω_{y} und ohne Phasenverschiebung (ϕ=0 zwischen den Auslenkungsschwingungen erfolgt (eine detailliertere Beschreibung erfolgt später mit Bezug zur Figur 3).

Das Scannsteuermodul 70 umfasst zudem eine Slowscannersteuerung zum Steuern der Ablenkung der Femtosekundenlaserpulse um die Scannachsen 31, 32 des Slow-Scannersystems 3. Die Slowscannersteuerung ist beispielsweise eingerichtet, die Ablenkung um die Scannachsen 31, 32 gemäss definierten Bearbeitungslinien s zu steuern, welche durch unterschiedliche Bearbeitungsmuster definiert sind und beispielsweise eine zugeordnete eindeutige Muster- respektive Linienkennung aufweisen. Um die Femtosekundenlaserpulse auf eine Bearbeitungslinie s abzulenken, die beispielsweise durch ein gespeichertes Bearbeitungsmuster oder eine Zeitfunktion definiert ist, verwendet das Steuermodul 7 entsprechende Steuerparameter zur Steuerung des Slow-Scannersystems 3, die der betreffenden Bearbeitungslinie s zugeordnet sind.

Das Rotationssteuermodul 90 ist Teil des Rotationssystems und ist eingerichtet die Drehung der Ablenkungsebene FF respektive der Scannbewegung f durch das Rotationsmodul 9, 9', 9" basierend auf dem Richtungsverlauf der Bearbeitungslinie s zu definieren und zu steuern. Das heisst das Rotationssteuermodul 90 und das Scannsteuermodul 70, insbesondere die Slowscannersteuerung, führen eine gekoppelte Steuerung des Slow-Scannersystems 3 und des Rotationsmoduls 9, 9', 9" aus, um die Bearbeitungslinie s zu Definieren und zu Steuern, und davon abhängig die Ausrichtung θ der Ablenkungsebene FF und damit der Scannbewegung f zu Bestimmen und zu Steuern. Je nach Ausführungsvariante des Rotationsmoduls 9, 9', 9" liefert das Rotationssteuermodul 90 von der aktuellen Richtung der Bearbeitungslinie s abhängige Steuersignale für das Rotationsmodul 9, 9', 9" an das Antriebsmodul, das Rotationselement und/oder das Fast-Scannersystem 5 (respektive die Fastscannersteuerung), um die Ablenkungsebene FF so um die optische Projektionsachse z zu drehen, dass sie einen definierten Ausrichtungswinkel zur Bearbeitungslinie s aufweist, beispielsweise π/2 oder einen anderen definierten Winkel.

Die Ausführungsvariante, in der die Funktion des Rotationsmoduls 9" durch das Fast-Scannersystem 5 respektive den/die um die zwei Scannachsen 51, 52 drehbaren Ablenkspiegel des Fast-Scannersystems 5 ausgeführt wird, hat den Vorteil, dass keine zusätzlichen Antriebselemente und/oder Rotationselemente in die Vorrichtung 1 aufgenommen werden müssen. Das Rotationssteuermodul 90 kann in diesem Fall in die Fastscannersteuerung integriert sein oder das Fast-Scannersystems 5 über Funktionsaufrufe der Fastscannersteuerung steuern.

Figur 2 zeigt ein Beispiel einer Scanntrajektorie t, bei welcher die überlagerte Scannbewegung f an eine Richtungsänderung der Bearbeitungslinie sₓ, s_{y}, beispielsweise von der x-Richtung auf die y-Richtung, angepasst wird. Wie in der Figur 2 schematisch dargestellt ist, ist das Rotationssteuermodul 90 eingerichtet, das Fast-Scannersystem 5 derart zu steuern, dass es die Auslenkungsamplitude B der Auslenkung b in y-Richtung um die Scannachse 51 und die Auslenkungsamplitude A der Auslenkung a in x-Richtung um die Scannachse 52 abhängig von der Richtung der Bearbeitungslinie sₓ, s_{y} setzt, beispielsweise so, dass bei der Bearbeitung durch das Slow-Scannersystem 3 entlang der Bearbeitungslinie sₓ in x-Richtung die Auslenkungsamplitude B des Fast-Scannersystems 5 auf eine definierte Scannbreite E und die Auslenkungsamplitude A des Fast-Scannersystems 5 auf Null gesetzt wird, und umgekehrt bei der Bearbeitung durch das Slow-Scannersystem 3 entlang der Bearbeitungslinie s_{y} in y-Richtung die Auslenkungsamplitude A des Fast-Scannersystems 5 auf die definierte Scannbreite F und die Auslenkungsamplitude B auf Null gesetzt wird. Im Übergangsbereich U von der Bearbeitung in x-Richtung auf die Bearbeitung in y-Richtung werden die Auslenkungsamplituden A, B der Auslenkungen a, b des Fast-Scannersystems 5 beispielsweise kontinuierlich erhöht respektive reduziert.

Figur 3 illustriert eine Sequenz von mehreren Zwischenstufen bei der Steuerung der Auslenkungen a, b um die Scannachsen 51, 52 zur Anpassung der Ausrichtung θ der Scannbewegung f von 0=π/2 bis θ=π an die Richtungsänderung von π/2 der Bearbeitungslinie sₓ, s_{y} im Übergangsbereich U von der x-Richtung in die y-Richtung (bei einem Betriebsmodus mit einer relativen Phase ϕ=π). Wie in der Figur 3 ersichtlich ist wird dabei die Auslenkungsamplitude A der Auslenkung a (in x-Richtung) um die Scannachse 52 (y-Achse) kontinuierlich von A=0 über A<B, A=B und A>B auf A=F erhöht, wohingegen die Auslenkungsamplitude B der Auslenkung b (in y-Richtung) um die Scannachse 51 (x-Achse) kontinuierlich von B=F über B>A, B=A und B<A auf B=0 reduziert wird.

In den Figuren 2, 4, 5 stellen die Kreise jeweils schematisch die Fokusmittelpunkte eines gepulsten Laserstrahls (Bearbeitungsstrahl L5) respektive eines Femtosekundenlaserpulses im Fokus Q dar. Die in der Figur 2 dargestellten Scanntrajektorien t weisen in bestimmten Bereichen ungleichmässig verteilte Laserpulse auf. Als Massnahme dagegen werden je nach Ausführungsvariante und/oder Anwendung der Laserstrahl respektive die Laserpulse durch das Filtermodul 4 ausserhalb eines definierten Durchlassbereichs respektive in einem bestimmten Ausblendbereich ausgeblendet, beispielsweise mittels eines Shutters und/oder einer Blende 41, wodurch selektiv bestimmte Bereiche der Scanntrajektorie t ausgewählt werden.

Das Blendensteuermodul 40 ist eingerichtet, den steuerbaren, variablen Blendenbereich respektive Durchlassbereich der Blende 41 im Filtermodul 4 zu steuern. Das Blendensteuermodul 40 ist beispielsweise eingerichtet, die Grösse des variablen Blendenbereichs respektive Durchlassbereichs abhängig von der Scanngeschwindigkeit des Slow-Scannersystems 3 zu steuern.

Im Beispiel der Figur 4 werden beispielsweise optional die Schwingungsspitzen, welche vergleichsweise dichter aufeinanderfolgende Laserpulse aufweisen, in den ausserhalb der definierten Scannbreite E liegenden Bereichen R1 durch das Filtermodul 4 ausgeblendet, vorzugsweise durch Blende 41, insbesondere eine Feldblende.

Im Beispiel der Figur 5, werden jeweils die aufsteigenden Flanken der Scanntrajektorie t im Bereich R2 ausgeblendet, so dass selektiv nur die absteigenden Äste der Scanntrajektorie t zur Bearbeitung verwendet werden, wobei beispielsweise zudem wie in Figur 4 auch die Schwingungsspitzen im Bereich R1 abgeschnitten werden. Zur Ausblendung des Bereichs R2 in der Scanntrajektorie steuert das Blendensteuermodul 40 den variablen Blendenbereich der Blende 41 so, dass die entsprechenden Femtosekundenlaserpulse im Bereich R2 in der überlagerten Scannbewegung f jeweils für aufsteigende Flanken ausgeblendet, jedoch für absteigende Flanken wieder durchgelassen werden, d.h. der Blendenbereich R2 wechselt synchronisiert mit der Schwingung der Scannbewegung f zwischen Ausblendemodus und Durchlassmodus. Vorzugsweise wird der Bereich R2 durch einen Shutter ausgeblendet der beim Laser angeordnet und der Strahlquelle 6 nachgeschaltet ist und zum Ausblenden des Bereichs R2 geschlossen und andernfalls geöffnet wird.

Um den Pulsabstand innerhalb eines Astes respektive einer Flanke zu anzugleichen, wird in einer weiteren Ausführungsvariante durch das Steuermodul 7 die Pulsfrequenz abhängig von der Position in der Scanntrajektorie t angepasst werden (z.B. über Pulspicker) oder die Auslenkungsamplitude A, B des Fast-Scannersystems 5 dynamisch verändert. Das Scannsteuermodul 70 ist beispielsweise eingerichtet, die Auslenkungsamplitude F der Scannbewegung f des Fast-Scannersystems 5 abhängig vom Richtungsverlauf der Bearbeitungslinie s und/oder abhängig von der aktuellen Scanngeschwindigkeit des Slow-Scannersystem 3 zu steuern. Durch die resultierende dynamische Vergrösserung (Streckung) respektive Verkleinerung (Stauchung) der Auslenkungsamplitude F der Scannkurve f kann der Abstand zwischen nacheinander folgenden einzelnen Femtosekundenlaserpulsen auf der Scannbewegung f respektive Scanntrajektorie t vergrössert respektive verkleinert werden und dadurch die Anzahl Laserpulse im Durchlassbereich, innerhalb der nicht ausgeblendeten Scannbreite E, variabel gesteuert werden.

Abschliessend soll angeführt werden, dass in der Beschreibung zwar Computerprogrammcode spezifischen funktionalen Modulen zugeordnet wurde, dass der Fachmann jedoch verstehen wird, dass der Computerprogrammcode unterschiedlich strukturiert geändert werden kann, ohne dabei vom Schutzgegenstand abzuweichen.

## Patentansprüche

1. Ophthalmologische Vorrichtung (1) zum Bearbeiten von Augengewebe (8) mittels Femtosekundenlaserpulsen, umfassend:
eine Projektionsoptik (2) zur fokussierten Projektion der Femtosekundenlaserpulse ins Augengewebe (8),
ein erstes der Projektionsoptik (2) vorgelagertes strahlablenkendes Scannersystem (3) mit einem oder zwei verkippbaren Ablenkspiegeln zum Scannen des Augengewebes (8) mit den Femtosekundenlaserpulsen entlang einer Bearbeitungslinie (s),
ein zweites dem ersten Scannersystem (3) vorgelagertes strahlablenkendes Scannersystem (5) zum Scannen des Augengewebes (8) mit den Femtosekundenlaserpulsen in einer der Bearbeitungslinie (s) überlagerten, in einer Ablenkungsebene (FF) verlaufenden Scannbewegung (f), und
ein Rotationssystem mit einem Rotationsmodul (9, 9', 9") zum Ausrichten der Ablenkungsebene (FF) durch eine definierte Drehung der Ablenkungsebene (FF) um eine optische Projektionsachse (z), **dadurch gekennzeichnet,**
**dass** das Rotationssystem ein Rotationssteuermodul (90) umfasst, das eingerichtet ist, die Drehung der Ablenkungsebene (FF) basierend auf dem Richtungsverlauf der Bearbeitungslinie (s) zu definieren und das Rotationsmodul (9, 9', 9") so zu steuern, dass die Ablenkungsebene (FF) mit einem definierten Winkel zur Bearbeitungslinie (s) ausgerichtet ist.

2. Vorrichtung (1) nach Anspruch 1, **dadurch gekennzeichnet, dass** das erste Scannersystem (3) zwei orthogonal zueinander ausgerichtete Scannachsen (31, 32) zum Ablenken der Femtosekundenlaserpulse aufweist, dass das zweite Scannersystem (5) eingerichtet ist, die Femtosekundenlaserpulse mit einer Scanngeschwindigkeit abzulenken, die ein Vielfaches der Scanngeschwindigkeit des ersten Scannersystems (3) beträgt, und dass das Rotationssystem dem ersten Scannersystem (3) vorgelagert ist.

3. Vorrichtung (1) nach Anspruch 1, **dadurch gekennzeichnet, dass** das Rotationsmodul (9, 9', 9") eines umfasst aus: ein aus Spiegeln ausgestaltetes Rotationselement, ein aus Prismen ausgestaltetes Rotationselement, und ein mit dem zweiten Scannersystem (5) gekoppeltes Antriebsmodul, welches eingerichtet ist das zweite Scannersystem (5) um die optische Projektionsachse (z) zu drehen.

4. Vorrichtung (1) nach Anspruch 1, **dadurch gekennzeichnet, dass** das zweite Scannersystem (5) zwei orthogonal zueinander ausgerichtete Scannachsen (51, 52) zum Ablenken der Femtosekundenlaserpulse aufweist, und dass das Rotationssteuermodul (90) eingerichtet ist, die Ausrichtung der in der Ablenkungsebene (FF) verlaufenden Scannbewegung (f) zur Bearbeitungslinie (s) durch gekoppelte Steuerung von der Auslenkungsamplitude (B) der Ablenkung (b) um die erste Scannachse (51) und von der Auslenkungsamplitude (A) der Ablenkung (a) um die zweite Scannachse (52) zu bestimmen.

5. Vorrichtung (1) nach einem der Ansprüche 1 bis 4, **gekennzeichnet durch** ein Scannsteuermodul (70), das eingerichtet ist, die Auslenkungsamplitude (F) der Scannbewegung (f) des zweiten Scannersystem (5) abhängig von mindestens einem aus: Richtungsverlauf der Bearbeitungslinie (s) und aktuelle Scanngeschwindigkeit des ersten Scannersystems (3) zu steuern.

6. Vorrichtung (1) nach einem der Ansprüche 1 bis 5, **gekennzeichnet durch** eine zwischen dem ersten Scannersystem (3) und dem zweiten Scannersystem (5) angeordnete Blende (41) zum Ausblenden von Femtosekundenlaserpulsen, die vom vorgelagerten zweiten Scannersystem (5) in einen Bereich (R1) ausserhalb eines definierten Durchlassbereichs (E) abgelenkt werden.

7. Vorrichtung (1) nach Anspruch 6, **gekennzeichnet durch** ein Scannsteuermodul (70), das eingerichtet ist, die Auslenkungsamplitude (F) der Scannbewegung (f) des zweiten Scannersystem (5) so zu steuern, dass der Abstand zwischen zwei aufeinanderfolgenden Femtosekundenlaserpulsen und die Anzahl der Femtosekundenlaserpulse im Durchlassbereich (E) variierbar ist.

8. Vorrichtung (1) nach einem der Ansprüche 6 oder 7, **dadurch gekennzeichnet, dass** die Blende (41) einen steuerbaren, variablen Blendenbereich respektive Durchlassbereich aufweist.

9. Vorrichtung (1) nach Anspruch 8, **gekennzeichnet durch** ein Blendensteuermodul (40), das eingerichtet ist, die Grösse des variablen Blendenbereichs respektive Durchlassbereichs abhängig von der Scanngeschwindigkeit des ersten Scannersystems (3) zu steuern.

10. Vorrichtung (1) nach einem der Ansprüche 6 bis 9, **dadurch gekennzeichnet, dass** die Blende (41) als Feldblende ausgestaltet ist.

11. Vorrichtung (1) nach einem der Ansprüche 1 bis 10, **dadurch gekennzeichnet, dass** das erste Scannersystem (3) einen im Vergleich zum zweiten Scannersystem (5) wesentlich grösseren Auslenkungsgrad aufweist.

12. Vorrichtung (1) nach einem der Ansprüche 2 bis 11, **dadurch gekennzeichnet, dass** die zwei orthogonal zueinander ausgerichteten Scannachsen (31, 32) des ersten Scannersystems (3) mit einem gemeinsamen Ablenkspiegel gekoppelt sind.

13. Vorrichtung (1) nach einem der Ansprüche 1 bis 12, **dadurch gekennzeichnet, dass** das zweite Scannersystem (5) eingerichtet ist, das Augengewebe (8) mit den Femtosekundenlaserpulsen in einer oszillierenden Scannbewegung (f) zu scannen.

14. Vorrichtung (1) nach einem der Ansprüche 1 bis 13, **gekennzeichnet durch** ein steuerbares Filtermodul (4) zum selektiven Ausschliessen von Femtosekundenlaserpulsen in einem definierten Bereich (R2) der überlagerten Scannbewegung (f).

## Claims

1. Ophthalmological device (1) for processing eye tissue (8) by means of femtosecond laser pulses comprising:
a projection optical unit (2) for the focused projection of the femtosecond laser pulses into the eye tissue (8),
a first beam-deflecting scanner system (3), disposed upstream of the projection optical unit (2), with one or two tiltable deflection mirrors for scanning the eye tissue (8) with the femtosecond laser pulses along a processing line (s),
a second beam-deflecting scanner system (5), disposed upstream of the first scanner system (3), for scanning the eye tissue (8) with the femtosecond laser pulses in a scanning movement (f) superimposed on the processing line (s), and running in a deflection plane (FF), and
a rotation system with a rotation module (9, 9', 9") for aligning the deflection plane (FF) by a defined rotation of the deflection plane (FF) about an optical projection axis (z), **characterized in that** the rotation system comprises a rotation control module (90), which is designed to define the rotation of the deflection plane (FF) on the basis of the directional course of the processing line (s) and to control the rotation module (9, 9', 9") in such a way that the deflection plane (FF) is aligned with a defined angle with respect to the processing line (s).

2. Device (1) according to Claim 1, **characterized in that** the first scanner system (3) has two scanning axes (31, 32) aligned orthogonally with respect to one another for deflecting the femtosecond laser pulses, **in that** the second scanner system (5) is designed to deflect the femtosecond laser pulses with a scanning speed that is a multiple of the scanning speed of the first scanner system (3), and **in that** the rotation system is disposed upstream of the first scanner system (3).

3. Device (1) according to Claim 1, **characterized in that** the rotation module (9, 9', 9") comprises one from: a rotation element configured from mirrors, a rotation element configured from prisms, and a drive module coupled to the second scanner system (5) and designed to rotate the second scanner system (5) about the optical projection axis (z).

4. Device (1) according to Claim 1, **characterized in that** the second scanner system (5) has two scanning axes (51, 52) aligned orthogonally with respect to one another for deflecting the femtosecond laser pulses, and **in that** the rotation control module (90) is designed to determine the alignment of the scanning movement (f) running in the deflection plane (FF) with respect to the processing line (s) by coupled control of the excursion amplitude (B) of the deflection (b) about the first scanning axis (51) and of the excursion amplitude (A) of the deflection (a) about the second scanning axis (52).

5. Device (1) according to any of Claims 1 to 4, **characterized by** a scanning control module (70), which is designed to control the excursion amplitude (F) of the scanning movement (f) of the second scanner system (5) depending on at least one from: directional course of the processing line (s) and current scanning speed of the first scanner system (3).

6. Device (1) according to any of Claims 1 to 5, **characterized by** a diaphragm (41) arranged between the first scanner system (3) and the second scanner system (5) and serving for masking out femtosecond laser pulses that are deflected by the upstream second scanner system (5) into a region (R1) outside a defined transmission region (E).

7. Device (1) according to Claim 6, **characterized by** a scanning control module (70), which is designed to control the excursion amplitude (F) of the scanning movement (f) of the second scanner system (5) in such a way that the distance between two successive femtosecond laser pulses and the number of femtosecond laser pulses in the transmission region (E) are variable.

8. Device (1) according to either of Claims 6 and 7, **characterized in that** the diaphragm (41) has a controllable, variable diaphragm region or transmission region.

9. Device (1) according to Claim 8, **characterized by** a diaphragm control module (40), which is designed to control the size of the variable diaphragm region or transmission region depending on the scanning speed of the first scanner system (3).

10. Device (1) according to any of Claims 6 to 9, **characterized in that** the diaphragm (41) is configured as a field stop.

11. Device (1) according to any of Claims 1 to 10, **characterized in that** the first scanner system (3) has a significantly greater degree of excursion in comparison with the second scanner system (5).

12. Device (1) according to any of Claims 2 to 11, **characterized in that** the two scanning axes (31, 32) of the first scanner system (3) that are aligned orthogonally with respect to one another are coupled to a common deflection mirror.

13. Device (1) according to any of Claims 1 to 12, **characterized in that** the second scanner system (5) is designed to scan the eye tissue (8) with the femtosecond laser pulses in an oscillating scanning movement (f).

14. Device (1) according to any of Claims 1 to 13, **characterized by** a controllable filter module (4) for selectively excluding femtosecond laser pulses in a defined region (R2) of the superimposed scanning movement (f).

## Revendications

1. Appareil ophtalmologique (1) destiné à traiter le tissu oculaire (8) au moyen d'impulsions laser femtoseconde, comprenant :
une optique de projection (2) destinée à concentrer la projection des impulsions laser femtoseconde dans le tissu oculaire (8),
un premier système de balayage (3) de déviation du faisceau monté devant l'optique de projection (2), comprenant un ou deux miroirs de déviation basculants destinés au balayage du tissu oculaire (8) avec les impulsions laser femtoseconde le long d'une ligne de traitement (s),
un deuxième système de balayage (5) de déviation du faisceau monté devant le premier système de balayage (3), destiné au balayage du tissu oculaire (8) avec les impulsions laser femtoseconde dans un mouvement de balayage (f) qui se déroule dans un plan de déviation (FF) superposé à la ligne de traitement (s), et
un système de rotation muni d'un module de rotation (9, 9', 9") destiné à l'orientation du plan de déviation (FF) par une rotation définie du plan de déviation (FF) autour d'un axe de projection optique (z), **caractérisé en ce que** le système de rotation comprend un module de commande de rotation (90) qui est conçu pour définir la rotation du plan de déviation (FF) en se basant sur le tracé directionnel de la ligne de traitement (s) et pour commander le module de rotation (9, 9', 9") de telle sorte que le plan de déviation (FF) est orienté avec un angle défini par rapport à la ligne de traitement (s).

2. Appareil (1) selon la revendication 1, **caractérisé en ce que** le premier système de balayage (3) possède deux axes de balayage (31, 32) orientés orthogonalement l'un par rapport à l'autre pour la déviation des impulsions laser femtoseconde, **en ce que** le deuxième système de balayage (5) est conçu pour dévier les impulsions laser femtoseconde avec une vitesse de balayage qui est égale à un multiple de la vitesse de balayage du premier système de balayage (3), et **en ce que** le système de rotation est monté devant le premier système de balayage (3).

3. Appareil (1) selon la revendication 1, **caractérisé en ce que** le module de rotation (9, 9', 9") comprend l'un des éléments suivants : un élément rotatif constitué de miroirs, un élément rotatif constitué de prismes et un module d'entraînement couplé au deuxième système de balayage (5), lequel est conçu pour faire tourner le deuxième système de balayage (5) autour de l'axe de projection optique (z).

4. Appareil (1) selon la revendication 1, **caractérisé en ce que** le deuxième système de balayage (5) possède deux axes de balayage (51, 52) orientés orthogonalement l'un par rapport à l'autre pour la déviation des impulsions laser femtoseconde, **en ce que** le module de commande de rotation (90) est conçu pour définir l'orientation du mouvement de balayage (f) se déroulant dans le plan de déviation (FF) par rapport à la ligne de traitement (s) par une commande couplée de l'amplitude de déviation (B) de la déviation (b) autour du premier axe de balayage (51) et de l'amplitude de déviation (A) de la déviation (a) autour du deuxième axe de balayage (52).

5. Appareil (1) selon l'une des revendications 1 à 4, **caractérisé par** un module de commande de balayage (70) qui est conçu pour commander l'amplitude de déviation (F) du mouvement de balayage (f) du deuxième système de balayage (5) en fonction d'au moins l'un parmi : tracé directionnel de la ligne de traitement (s) et vitesse de balayage actuelle du premier système de balayage (3).

6. Appareil (1) selon l'une des revendications 1 à 5, **caractérisé par** un obturateur (41) disposé entre le premier système de balayage (3) et le deuxième système de balayage (5) pour l'obturation des impulsions laser femtoseconde qui sont déviées par le deuxième système de balayage (5) placé à l'avant dans une zone (R1) en-dehors d'une zone de passage (E) définie.

7. Appareil (1) selon la revendication 6, **caractérisé par** un module de commande de balayage (70) qui est conçu pour commander l'amplitude de déviation (F) du mouvement de balayage (f) du deuxième système de balayage (5) de telle sorte que l'écart entre deux impulsions laser femtoseconde successives et le nombre d'impulsions laser femtoseconde dans la zone de passage (E) est variable.

8. Appareil (1) selon l'une des revendications 6 ou 7, **caractérisé en ce que** l'obturateur (41) présente une zone d'obturation et une zone de passage correspondante commandable variable.

9. Appareil (1) selon la revendication 8, **caractérisé par** un module de commande d'obturateur (40) qui est conçu pour commander la taille de la zone d'obturation et de la zone de passage correspondante variable en fonction de la vitesse de balayage du premier système de balayage (3).

10. Appareil (1) selon l'une des revendications 6 à 9, **caractérisé en ce que** l'obturateur (41) est réalisé sous la forme d'un obturateur de champ.

11. Appareil (1) selon l'une des revendications 1 à 10, **caractérisé en ce que** le premier système de balayage (3) présente un degré de déviation nettement plus grand en comparaison du deuxième système de balayage (5).

12. Appareil (1) selon l'une des revendications 2 à 11, **caractérisé en ce que** les deux axes de balayage (31, 32) orientés orthogonalement l'un par rapport à l'autre du premier système de balayage (3) sont couplés avec un miroir de déviation commun.

13. Appareil (1) selon l'une des revendications 1 à 12, **caractérisé en ce que** le deuxième système de balayage (5) est conçu pour balayer le tissu oculaire (8) avec les impulsions laser femtoseconde dans un mouvement de balayage (f) oscillant.

14. Appareil (1) selon l'une des revendications 1 à 13, **caractérisé par** un module filtrant commandable (4) destiné à l'exclusion sélective d'impulsions laser femtoseconde dans une zone définie (R2) du mouvement de balayage (f) superposé.
